(19) Europäisches Patentamt
European Patent Office
Office européen des brevets

(11) Veröffentlichungsnummer : **0 084 834**
**B1**

(12) # EUROPÄISCHE PATENTSCHRIFT

(45) Veröffentlichungstag der Patentschrift : **21.01.87**

(51) Int. Cl.⁴ : **C 07 D249/08**, C 07 D233/60, A 01 N 43/64, A 01 N 43/50// C07D303/32, C07D405/06

(21) Anmeldenummer : **83100335.5**

(22) Anmeldetag : **17.01.83**

(54) **Substituierte 1-Hydroxyalkyl-azolyl-Derivate, Verfahren zu ihrer Herstellung sowie ihre Verwendung als Fungizide und Pflanzenwachstumsregulatoren.**

(30) Priorität : **27.01.82 DE 3202601**

(43) Veröffentlichungstag der Anmeldung : **03.08.83 Patentblatt 83/31**

(45) Bekanntmachung des Hinweises auf die Patenterteilung : **21.01.87 Patentblatt 87/04**

(84) Benannte Vertragsstaaten : **AT BE CH DE FR GB IT LI NL SE**

(56) Entgegenhaltungen :
**EP-A- 0 019 134**
**EP-A- 0 040 345**
**EP-A- 0 052 424**
**EP-A- 0 054 974**
**EP-A- 0 057 357**
**EP-A- 0 061 051**
**EP-A- 0 061 835**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Holmwood, Graham, Dr.**
**Katernberger Strasse 184**
**D-5600 Wuppertal 1 (DE)**
Erfinder : **Regel, Erik, Dipl.-Ing.**
**Untere Bergerheide 26**
**D-5600 Wuppertal 1 (DE)**
Erfinder : **Jäger, Gerhard, Dr.**
**Gellertstrasse 18**
**D-5090 Leverkusen (DE)**
Erfinder : **Büchel, Karl Heinz, Prof. Dr.**
**Dabringhausener Strasse 42**
**D-5093 Burscheid (DE)**
Erfinder : **Lürssen, Klaus, Dr.**
**August-Kierspel-Strasse 89**
**D-5060 Bergisch-Gladbach 2 (DE)**
Erfinder : **Frohberger, Paul-Ernst, Dr.**
**Willi-Baumeister-Strasse 5**
**D-5090 Leverkusen 1 (DE)**
Erfinder : **Brandes, Wilhelm, Dr.**
**Eichendorffstrasse 3**
**D-5653 Leichlingen (DE)**
Erfinder : **Paul, Volker, Prof. Dr.**
**Ahornstrasse 5**
**D-5650 Solingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 084 834 B1**

**Beschreibung**

Die vorliegende Erfindung betrifft neue substituierte 1-Hydroxyalkylazolyl-Derivate, mehrere Verfahren zu ihrer Herstellung sowie ihre Verwendung als Fungizide und Pflanzenwachstumsregulatoren.

Es ist bereits bekannt geworden, daß bestimmte Triazolylalkanone und -ole, wie beispielsweise 1-Phenyl- oder 1-(4-Chlorphenyl)- oder 1-(2,4-Dichlorphenyl)-2-(1,2,4-triazol-1-yl)-1-ethanol sowie 2,2-Dimethyl-4-(1,2,4-triazol-1-yl)-3-dodecanon und ω-Phenyl-ω-(1,2,4-triazol-1-yl)-acetophenon, fungizide Eigenschaften aufweisen (vergleiche DE-A 24 31 407). Die Wirksamkeit dieser Verbindungen ist jedoch, insbesondere bei niedrigen Aufwandmengen und -konzentrationen, nicht immer ganz befriedigend. Ferner sind Ether-Derivate von bestimmten Hydroxyalkyl-imidazolen aus der Patentliteratur bekannt (vgl. US-A 4 123 542 und 4 277 475), die eine Wirkung gegen humanpathogene Pilze aufweisen und als Arzneimittel und zudem auch als Contrazeptiva verwendet werden können (US-A 4 277 475).

Es ist weiterhin bekannt, daß 1-(4-Chlorphenoxy)-3,3-dimethyl-1-(1,2,4-triazol-1-yl)-2-butanon gute fungizide Wirksamkeit aufweist (vergleiche DE-B 22 01 063. Die Wirkung dieser Verbindung ist jedoch in bestimmten Indikationsbereichen, wie insbesondere bei Reiskrankheiten, vor allem bei niedrigen Aufwandmengen, nicht immer ausreichend.

Ferner ist bereits bekannt geworden daß bestimmte Biphenyl-yl-hydroxyalkyl-triazolyl-Derivate, wie beispielsweise 2-(4-Biphenylyl)-1-phenyl-3-(1,2,4-triazol-1-yl)-2-propanol und 1-(4-Biphenylyl)-1-(2-chlor-bzw. fluor-phenyl)-2-(1,2,4-triazol-1-yl)-1-ethanol, allgemein gute fungizide Eigenschaften aufweisen und in entsprechenden Aufwandmengen auch pflanzenwachstumsregulierende Wirkung besitzen. Die Wirksamkeit dieser Verbindungen ist jedoch, insbesondere bei niedrigen Aufwandmengen und -konzentrationen, nicht immer befriedigend.

Außerdem werden in der EP-A 0 040 345 bestimmte fungizid und pflanzenwuchsregulierend wirksame 1-Hydroxyethyl-azol-Derivate beschrieben, in denen das Kohlenstoffatom, das die Hydroxygruppe trägt, unter anderem mit einem Alkylrest mit vorzugsweise 1 bis 4 Kohlenstoffatomen, mit einem gegebenenfalls substituierten Cycloalkyl-Rest oder mit einem gegebenenfalls substituierten Phenyl-Rest verbunden sein kann.

Schließlich sind aus der EP-A 0 054 974 und der EP-A 0 061 835 1-Hydroxyethyl-azol-Derivate bekannt, in denen das Kohlenstoffatom, das die Hydroxygruppe trägt, mit einem Alkyl-Rest, mit einem gegebenenfalls substituierten Cycloalkylrest oder mit einem gegebenenfalls substituierten Phenyl-Rest verknüpft sein kann. Entsprechende Verbindungen, in denen das Carbinol-Kohlenstoffatom einen substituierten Alkylrest bzw. einen verzweigten Alkylrest mit mehr als 4 Kohlenstoffatomen trägt, werden jedoch nicht offenbart.

Es wurden neue substituierte 1-Hydroxyalkyl-azolyl-Derivate der allgemeinen Formel

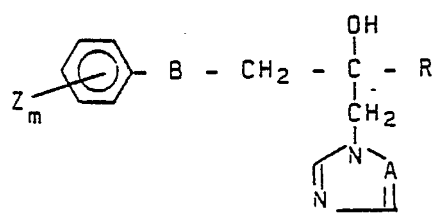

(I)

in welcher

A für ein Stickstoffatom oder die CH-Gruppe steht,

B für Sauerstoff, Schwefel oder die $CH_2$-Gruppe steht,

R für die Gruppierungen

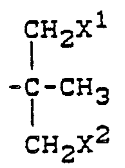 und 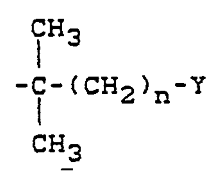

steht, wobei

$X^1$ für Wasserstoff, Fluor, Chlor oder Brom steht,

$X^2$ für Fluor, Chlor oder Brom steht,

Y für Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für gegebenenfalls substituiertes Phenyl, Phenoxy, Phenylthio, Phenylmethoxy und Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl,

n für die Zahlen 0, 1 oder 2 steht,

Z für Fluor, Chlor, Brom, Methyl, tert.-Butyl, Cyclohexyl, Methoxy, Methylthio, Trifluormethyl, Trifluormethoxy, Trifluormethylthio sowie für gegebenenfalls durch Fluor, Chlor und Methyl substituiertes Phenyl, Phenoxy, Benzyl oder Benzyloxy steht, und

m für die Zahlen 0, 1, 2 oder 3 steht,

sowie deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen.

Weiterhin wurde gefunden, daß man die substituierten 1-Hydroxyalkyl-azolyl-Derivate der Formel (I) sowie deren Säureadditions-Salze und Metallsalz-Komplexe erhält, wenn man

a) Oxirane der Formel

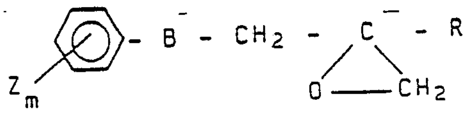

(II)

in welcher B, R, Z und m die oben angegebene Bedeutung haben, mit Azolen der Formel

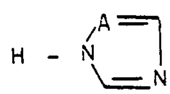

(III)

in welcher A die oben angegebene Bedeutung hat, in Gegenwart eines Verdünnungsmittels und gegebenenfalls in Gegenwart einer Base umsetzt, oder

b) Azolylmethyl-oxirane der Formel

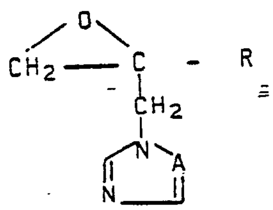

(IV)

in welcher A und R die oben angegebene Bedeutung haben, mit (Thio)Phenolen der Formel

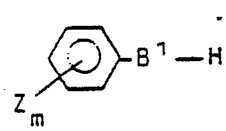

(V)

in welcher

Z und m die oben angegebene Bedeutung haben und

$B^1$ für Sauerstoff oder Schwefel steht,

in Gegenwart eines Verdünnungsmittels und gegebenenfalls in Gegenwart einer Base umsetzt, und gegebenenfalls anschließend an die so erhaltenen Verbindungen der Formel (I) eine Säure oder ein Metallsalz addiert.

Außerdem wurde gefunden, daß die neuen substituierten 1-Hydroxyalkyl-azolyl-Derivate der Formel (I) sowie deren Säureadditions-Salze und Metallsalz-Komplexe starke fungizide und pflanzenwachstumsregulierende Eigenschaften besitzen.

Überraschenderweise zeigen die erfindungsgemäßen Stoffe bessere fungizide und pflanzenwachstumsregulierende Wirkungen als die oben genannten, aus dem Stand der Technik bekannten Triazolyl-Derivate.

Die erfindungsgemäßen substituierten 1-Hydroxyalkylazolyl-Derivate sind durch die Formel (I) allgemein definiert. Sie unterscheiden sich von denjenigen Verbindungen, die in der EP-A 0 040 345, der EP-A 0 054 974 und der EP-A 0 061 835 beschrieben werden, in charakteristischer Weise durch die Bedeutungen der an das Carbinol-Kohlenstoffatom gebundenen Substituenten.

Zu den Säuren, die an die substituierten 1-Hydroxyalkylazolyl-Derivate der Formel (I) addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronenensäure, Salizylsäure, Sorbinsäure und Milchsäure,

sowie Sulfonsäuren, wie z. B. p-Toluolsulfonsäure und 1,5-Naphthalindisulfonsäure.

Bevorzugte Metallsalz-Komplexe von substituierten 1-Hydroxyalkyl-azolyl-Derivaten der Formel (I) sind Additionsprodukte aus Salzen von Metallen der II. bis IV. Haupt- und der I. und II. sowie IV. bis VIII. Nebengruppe und denjenigen substituierten 1-Hydroxyalkyl-azolyl-Derivaten der Formel (I), in denen die Substituenten A, B, R und $Z_m$ die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z. B. die Chlorwasserstoffsäure und die Bromwasserstoffsäure, ferner Phosphorsäure, Salpetersäure und Schwefelsäure.

Verwendet man beispielsweise 2-(4-Fluorphenoxymethyl)-2-(fluor-tert.-butyl)-oxiran und 1,2,4-Triazol als Ausgangsstoffe, so kann der Ablauf des erfindungsgemäßen Verfahrens (a) durch das folgende Formelschema wiedergegeben werden :

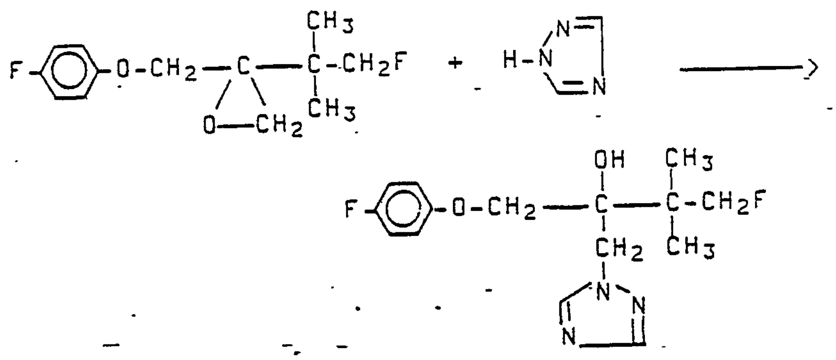

Verwendet man beispielsweise 2-(Methoxy-tert.-butyl)-2-(1,2,4-triazol-1-yl-methyl)-oxiran und 4-Chlorphenol als Ausgangsstoffe, so kann der Ablauf des erfindungsgemäßen Verfahrens (b) durch das folgende Formelschema wiedergegeben werden :

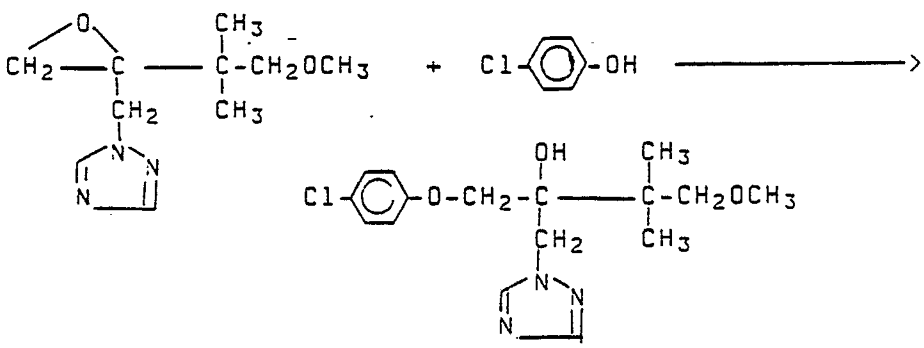

Die bei der Durchführung des erfindungsgemäßen Verfahrens (a) als Ausgangsstoffe zu verwendenden Oxirane sind durch die Formel (II) allgemein definiert. In dieser Formel haben B, R, Z und der Index m die Bedeutungen, die bereits im Zusammenhang mit der Beschreibung der erfindungsgemäßen Stoffe der Formel (I) für diese Substituenten bzw. für den Index m genannt wurden.

Die Oxirane der Formel (II) sind noch nicht bekannt. Sie stellen interessante Zwischenprodukte dar und können in allgemein bekannter Art und Weise erhalten werden, indem man Ketone der Formel

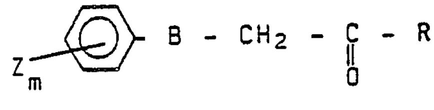

(VI)

in welcher B, R, Z und m die oben angegebene Bedeutung haben, entweder

α) mit Dimethyloxosulfonium-methylid der Formel

$$\overset{\delta+\ \ \delta-}{(CH_3)_2SOCH_2} \qquad (VII)$$

in Gegenwart eines Verdünnungsmittels umsetzt, oder

β) mit Trimethylsulfonium-methylsulfat der Formel

$$\left[(CH_3)_3\overset{(+)}{S}\right]\ \ CH_3\overset{(-)}{SO_4} \qquad (VIII)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (VI) sind bekannt (vergl. z. B. DE-A 26 32 603, DE-A 26 32 602, DE-A 26 35 664, DE-A 26 35 666, DE-A 29 18 894, DE-A 29 18 893, DE-C 22 01 063, DE-A 27 05 678, DE-A 27 37 489), bzw. sind sie Gegenstand eigener älterer Patentanmeldungen (vergl. DE-A 30 21 551, DE-A 31 19 390 und DE-A 31 01 143), bzw. lassen sie sich nach im Prinzip bekannten Verfahren herstellen.

Das bei der Verfahrensvariante (α) benötigte Dimethyloxosulfoniummethylid der Formel (VII) ist ebenfalls bekannt (vergleiche J. Amer. Chem. Soc. *87,* 1363-1364 (1965)). Es wird bei der obigen Umsetzung in frisch zubereitetem Zustand verarbeitet, indem man es *in situ* durch Umsetzung von Trimethyloxosulfoniumjodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (VIII) ist ebenfalls bekannt (vgl. Heterocycles *8,* 397 (1977)). Es wird bei der obigen Umsetzung ebenfalls in frisch hergestelltem Zustand eingesetzt, indem man es durch Reaktion von Dimethylsulfid mit Dimethylsulfat *in situ* erzeugt.

Bei der Variante (α) des Verfahrens zur Herstellung der Oxirane der Formel (II) kommt als Verdünnungsmittel vorzugsweise Dimethylsulfoxid infrage.

Die Reaktionstemperaturen können bei der oben beschriebenen Verfahrensvariante (α) innerhalb eines größeren Bereiches variiert werden. Im allgemeinen arbeitet man bei Temperaturen zwischen 20 °C und 80 °C.

Die Durchführung des Verfahrens zur Herstellung der Oxirane der Formel (II) nach der Variante (α) und die Aufarbeitung des bei dieser Synthese anfallenden Reaktionsgemisches erfolgen nach üblichen Methoden (vgl. J. Amer. Chem. Soc. *87,* 1363-1364 (1965)).

Bei der Variante (β) zur Herstellung der Oxirane der Formel (II) kommt als inertes organisches Lösungsmittel vorzugsweise Acetonitril in Betracht.

Als Basen können bei der Verfahrensvariante (β) starke anorganische oder organische Basen verwendet werden. Vorzugsweise infrage kommt Natriummethylat.

Die Reaktionstemperaturen können bei der oben beschriebenen Verfahrensvariante (β) innerhalb eines bestimmten Bereiches variiert werden. Im allgemeinen arbeitet man bei Temperaturen zwischen 0 °C und 60 °C, vorzugsweise bei Raumtemperatur.

Die Durchführung des Verfahrens zur Herstellung der Oxirane der Formel (II) nach der Variante (β) und die Aufarbeitung des bei dieser Synthese anfallenden Reaktionsproduktes erfolgen nach üblichen Methoden (vgl. Heterocycles *8,* 397 (1977)).

Die Oxirane der Formel (II) können bei dem erfindungsgemäßen Verfahren gegebenenfalls ohne Isolierung direkt weiter umgesetzt werden.

Die außerdem für das erfindungsgemäße Verfahren (a) als Ausgangsstoffe zu verwendenden Azole sind durch die Formel (III) allgemein definiert. In dieser Formel steht A für die Bedeutungen, die bereits in der Erfindungsdefinition für diesen Substituenten genannt wurden.

Die Azole der Formel (III) sind allgemein bekannte Verbindungen der organischen Chemie.

Die bei der Durchführung des erfindungsgemäßen Verfahrens (b) als Ausgangsstoffe zu verwendenden Azolylmethyloxirane sind durch die Formel (IV) allgemein definiert. In dieser Formel haben A und R die Bedeutungen, die bereits im Zusammenhang mit der Beschreibung der erfindungsgemäßen Stoffe der Formel (I) für diese Substituenten genannt wurden.

Die Azolylmethyl-oxirane der Formel (IV) sind noch nicht bekannt. Sie sind jedoch teilweise Gegenstand einer eigenen älteren Anmeldung (vergl. DE-A 31 11 238), bzw. können sie in allgemein bekannter Art und Weise erhalten werden, indem man Azolo-ketone der Formel

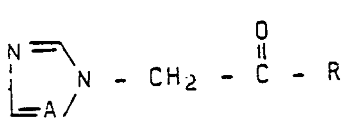

(IX)

in welcher A und R die oben angegebene Bedeutung haben, entsprechend den oben angegebenen Verfahrensvarianten (α) und (β) expoxidiert.

Die Azolo-ketone der Formel (IX) sind bekannt (vergl. DE-A 24 31 407, DE-A 26 38 470, DE-A 28 20 361) bzw. sind sie Gegenstand einer eigenen älteren Patentanmeldung (vergl. DE-A 30 48 266) bzw. lassen sie sich nach im Prinzip bekannten Verfahren herstellen.

Die außerdem für das erfindungsgemäße Verfahren als Ausgangsstoffe zu verwendenden (Thio)Phenole sind durch die Formel (V) allgemein definiert. In dieser Formel stehen Z und der Index m für die

Bedeutungen, die bereits im Zusammenhang mit der Beschreibung der erfindungsgemäßen Stoffe der Formel (I) für diesen Substituenten bzw. für den Index m genannt werden. $B^1$ steht für Sauerstoff oder Schwefel.

Die (Thio)Phenole der Formel (V) sind allgemein bekannte Verbindungen der organischen Chemie.

Als Verdünnungsmittel kommen für die erfindungsgemäßen Verfahren (a) und (b) unter den Reaktionsbedingungen inerte organische Lösungsmittel infrage. Hierzu gehören vorzugsweise Alkohole, wie z. B. Ethanol, Methoxyethanol oder Propanol ; Ketone, wie z. B. 2-Butanon ; Nitrile, wie z. B. Acetonitril ; Ester, wie z. B. Essigester ; Ether, wie z. B. Dioxan ; aromatische Kohlenwasserstoffe, wie z. B. Benzol und Toluol ; oder Amide, wie z. B. Dimethylformamid.

Als Basen kommen für die erfindungsgemäßen Umsetzungen alle üblicherweise verwendbaren anorganischen und organischen Basen in Betracht. Hierzu gehören vorzugsweise Alkalicarbonate, wie z. B. Natrium- und Kaliumcarbonat ; Alkalihydroxide, wie z. B. Natriumhydroxid ; Alkalialkoholate, wie z. B. Natrium- und Kalium-methylat und -ethylat ; Alkalihydride, wie z. B. Natriumhydrid ; sowie niedere tertiäre Alkylamine, Cycloalkylamine und Aralkylamine, wie insbesondere Triethylamin.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0 und 200 °C, vorzugsweise zwischen 60 und 150 °C.

Die erfindungsgemäßen Umsetzungen können gegebenenfalls unter erhöhtem Druck durchgeführt werden. Im allgemeinen arbeitet man zwischen 1 und 50 bar, vorzugsweise zwischen 1 und 25 bar.

Bei der Durchführung des erfindungsgemäßen Verfahrens (a) setzt man vorzugsweise auf 1 Mol Oxiran der Formel (II) 1 bis 2 Mol Azol und gegebenenfalls 1 bis 2 Mol Base ein ; bei der Durchführung des erfindungsgemäßen Verfahrens (b) setzt man vorzugsweise auf 1 Mol Azolylmethyl-oxiran der Formel (IV) 1 bis 2 Mol (Thio)Phenol der Formel (V) und gegebenenfalls 1 bis 2 Mol Base ein. Die Isolierung der Endprodukte erfolgt jeweils in allgemein üblicher Weise.

Die nach den erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditionssalzen der Verbindungen der Formel (I) kommen vorzugsweise folgende Säuren infrage : Die Halogenwasserstoffsäuren, wie z. B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure, Milchsäure sowie Sulfonsäuren wie z. B. p-Toluolsulfonsäure und 1,5-Naphthalindisulfonsäure.

Die Säureadditionssalze der Verbindungen der Formel (I) können in einer Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Hauptgruppe und der I. und II. sowie IV. bis VIII. Nebengruppe des Periodensystems infrage, wobei Kupfer, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metall-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, isolieren und gegebenenfalls durch Umkristallisation reinigen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auf mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens (« Lagerns ») der Pflanzen vor der Ernte

verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z. B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z. B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z. B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden (« Ausdünnung »), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z. B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z. B. Ananas oder Zierpflanzen, in Gärtnereien, zu einem Zeitpunkt keimen, austreiben oder blühen, an dem sie normalerweise hierzu keine Bereitschaft zeigen. Eine Verzögerung des Austreibes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg zur Bekämpfung solcher Pilze eingesetzt werden, die echte Mehltauerkrankungen hervorrufen ; so zur Bekämpfung von Erysiphe-Arten, wie z. B. gegen den Erreger des Gersten- bzw. des Getreidemehltaus (Erysiphe graminis) oder von Sphaerotheca-Arten, wie z. B. gegen den Erreger des Gurkenmehltaus (Sphaerotheca fuliginea) ; sowie auch zur Bekämpfung von Venturia-Arten, wie z. B. gegen den Erreger des Apfelschorfs (Venturia inaequalis) ; von Pellicularia sasakii am Reis und von Pyrenophora teres am Getreide.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln, Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid. Als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage : z. B. nichtionogene und anionische Emulgatoren, wie Polyoxyethylen-Fettsäureester, Polyoxyethylen-Fettalkoholether, z. B. Alkylarylpolyglycol-ether, Alkylsulfonate, Alkylsulfate, Arylsulfonate sowie Eiweißhydrolysate. Als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z. B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,000 1 Gew.-

%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Herstellungsbeispiele

### Beispiel 1

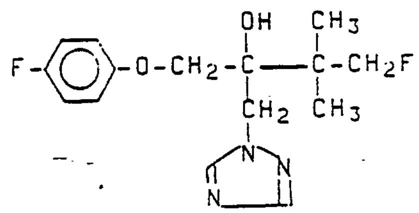

(Verfahren a)

0,46 g (0,02 Mol) Natrium in 200 ml n-Propanol werden mit 15,2 g (0,22 Mol) 1,2,4-Triazol versetzt. Man erhitzt die Lösung zum Sieden und versetzt tropfenweise mit 48,5 g (0,2 Mol) 2-(4-Fluorphenoxymethyl)-2-(fluor-tert.-butyl)-oxiran, gelöst in 50 ml n-Propanol. Man läßt die Reaktionslösung 48 Stunden unter Rückfluß nachrühren, engt ein und versetzt den Rückstand mit 200 ml Essigester und 100 ml Wasser. Die organische Phase wird abgetrennt, zweimal mit Wasser gewaschen, über Natriumsulfat getrocknet und eingeengt. Der Rückstand wird in Ether gelöst und mit Chlorwasserstoff begast. Der entstehende Niederschlag wird abgesaugt, mit Ether gewaschen und mit Essigester/1n-Natronlauge versetzt. Das anfallende Rohprodukt wird über eine Kieselgelsäule (Laufmittel : Methylenchlorid/Essigester, 2 : 1) gereinigt und aus Acetonitril umkristallisiert. Man erhält 17,9 g (29 % der Theorie) 4-Fluor-2-(4-fluorphenoxymethyl)-3,3-dimethyl-1-(1,2,4-triazol-1-yl)-butan-2-ol vom Schmelzpunkt 97-99 °C.

Herstellung des Ausgangsproduktes

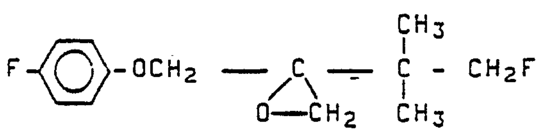

Eine Lösung von 47,3 ml (0,5 Mol) Dimethylsulfat in 250 ml absolutem Acetonitril wird bei Raumtemperatur mit einer Lösung von 40,5 ml (0,55 Mol) Dimethylsulfid in 50 ml absolutem Acetonitril versetzt. Man läßt die Reaktionsmischung 4 Tage bei Raumtemperatur rühren. Danach werden 55,1 g (0,24 Mol) 4-Fluor-1-(4-chlorphenoxy)-3,3-dimethyl-butan-2-on in 50 ml absolutem Acetonitril zugetropft und dann 30,4 g (0,56 Mol) Natriummethylat zugesetzt. Man läßt das Reaktionsgemisch 4 Tage nachrühren. Anschließend wird eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 49,1 g (85 % der Theorie) 2-(4-Fluorphenoxymethyl)-2-(fluor-tert.-butyl)-oxiran vom Siedepunkt 81-83 °C/0,09 mbar.

### Beispiel 2

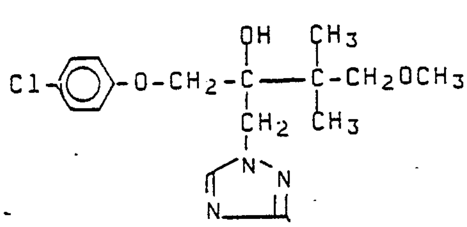

(Verfahren b)

8,1 g (0,063 3 Mol) 4-Chlorphenol werden zu einer Lösung von 0,32 g (0,014 Mol) Natrium in 100 ml absolutem Ethanol gegeben. Dazu werden 10,3 g (0,048 8 Mol) 2-(Methoxy-tert.-butyl)-2-(1,2,4-triazol-1-yl-methyl)-oxiran, gelöst in 300 ml absolutem Ethanol, gegeben und das Reaktionsgemisch 48 Stunden unter Rückfluß erhitzt. Man läßt abkühlen und engt das Reaktionsgemisch ein. Der Rückstand wird in

Essigester aufgenommen, einmal mit Wasser, einmal mit 1n-Natronlauge, erneut mit Wasser, danach mit 1n-Salzsäure und 1n-Natronlauge, wiederum mit Wasser und schließlich mit gesättigter Kochsalzlösung gewaschen. Die organische Phase wird über Natriumsulfat getrocknet und eingeengt. Man erhält 10,7 g (64,6 % der Theorie) 2-(4-Chlorphenoxymethyl)-3,3-dimethyl-4-methoxy-1-(1,2,4-triazol-1-yl)-butan-2-ol vom Brechungsindex $n_D^{20} = 1,531$.

Herstellung des Ausgangsproduktes

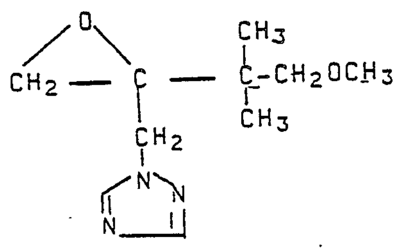

Unter Stickstoff-Atmosphäre werden 24 g (1 Mol) Natriumhydrid (80 %-ig in Paraffinöl) und 220 g (1 Mol) Trimethylsulfoxoniumiodid bei 10 °C gemischt und langsam mit 1 000 ml Dimethylsulfoxid versetzt. Nach einer Stunde werden in die Suspension 177,5 g (0,9 Mol) 3,3-Dimethyl-4-methoxy-1-(1,2,4-triazol-1-yl)-butan-2-on, gelöst in 200 ml Tetrahydrofuran, zugetropft.

Das Gemisch wird 2 Tage bei Raumtemperatur gerührt. Die Lösungsmittel werden im Hochvakuum abgezogen, der Rückstand mit Essigester verrührt und abgesaugt. Das Filtrat wird dreimal mit verdünnter Kochsalzlösung gewaschen, über Natriumsulfat getrocknet, eingeengt und der Rückstand wird im Hochvakuum destilliert. Man erhält 163 g (86 % der Theorie) 2-(Methoxy-tert.-butyl)-2-(1,2,4-triazol-1-yl-methyl)-oxiran vom Siedepunkt 89-92 °C/0,008 mbar.

Beispiel 3

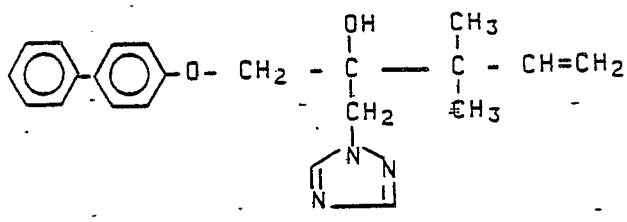

(Verfahren a)

Zu einer siedenden Lösung von 1 g (0,012 Mol) Natriumpropylat und 9,1 g (0,132 Mol) 1,2,4-Triazol in 50 ml n-Propanol werden 35,3 g (0,120 Mol) 2-(4-Biphenylyloxymethyl)-2-(3,3-dimethyl-1-propen-3-yl)-oxiran, gelöst in 80 ml n-Propanol, zugegeben. Nach zweitägigem Sieden unter Rückfluß wird die Lösung unter vermindertem Druck eingedampft, der ölige Rückstand in 200 ml Essigester aufgenommen und dreimal mit je 50 ml Wasser gewaschen. Nach dem Trocknen der organischen Phase über wasserfreiem Natriumsulfat wird die Lösung unter vermindertem Druck eingeengt. Nach Verreiben des verbleibenden Rückstandes mit 150 ml Essigester erhält man 6,6 g (15,1 % der Theorie) 4-(4-Biphenylyloxymethyl)-3,3-dimethyl-4-hydroxy-5-(1,2,4-triazol-4-yl)-1-penten als farblose Kristalle vom Schmelzpunkt 171-173 °C. Das nach dem Eindampfen des Filtrats verbleibende Oel (37,1 g) wird in 200 ml Aceton aufgenommen und mit einer Lösung von 34,6 g Naphthalindisulfonsäure-(1,5) in 100 ml Aceton versetzt. Das auskristallisierende Salz wird abgenutscht, mit Aceton gewaschen und in einem Gemisch von je 250 ml Wasser und Dichlormethan aufgenommen. Durch Einrühren von 10-%iger Natriumcarbonat-Lösung wird alkalisch gestellt. Nach dem Einengen der organischen Phase erhält man 24 g eines Oels, das in Essigester gelöst und anschließend über Kieselgel filtriert wird. Nach Einengen des Filtrats erhält man 17,8 g (40,8 % der Theorie) 4-(4-Biphenylyloxymethyl)-3,3-dimethyl-4-hydroxy-5-(1,2,4-triazol-1-yl)-1-penten in Form farbloser Kristalle vom Schmelzpunkt 85-87 °C.

Herstellung des Ausgangsproduktes

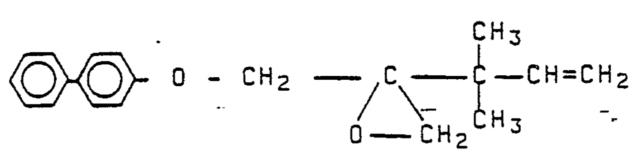

Zu einer Lösung von 49,4 ml (0,518 Mol) Dimethylsulfat in 250 ml Acetonitril läßt man unter Außenkühlung bei 25 °C im Laufe von 2 Stunden eine Lösung von 41,9 ml (0,567 Mol) Dimethylsulfid in 50 ml Acetonitril zutropfen. Nach viertägigem Stehen bei Raumtemperatur werden dann innerhalb 1 Stunde 87,4 g (0,31 Mol) 5-(4-Biphenylyloxy)-3,3-dimethyl-1-penten-4-on, gelöst in 90 ml Acetonitril, eingerührt. Anschließend werden bei 25 °C 31,6 g (0,58 Mol) Natriummethylat eingetragen. Nach zwölfstündigem Rühren bei Raumtemperatur wird vom ausgeschiedenen Salz abfiltriert und das Filtrat eingeengt. Der ölige Rückstand wird in 500 ml Essigester aufgenommen, die Lösung zweimal mit je 500 ml Wasser gewaschen und über wasserfreiem Natriumsulfat getrocknet. Nach dem Einengen im Vakuum werden 73,4 g (80,4 % der Theorie) 2-(4-Biphenylyloxymethyl)-2-(3,3-dimethyl-1-propen-3-yl)-oxiran als farblose, wachsartige Masse erhalten.

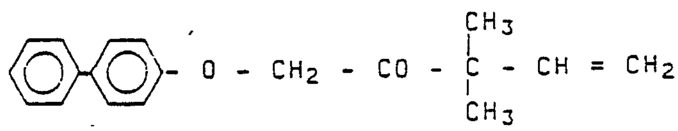

Zu einem Gemisch aus 184,9 g (1,34 Mol) Kaliumcarbonat und 227,8 g (1,34 Mol) 4-Hydroxybiphenyl in 1 000 ml siedendem Aceton werden innerhalb einer Stunde 196,5 g (1,34 Mol) 5-Chlor-3,3-dimethyl-1-penten-4-on zugetropft. Man erhitzt nach 5 Stunden zum Sieden, läßt das Gemisch auf Raumtemperatur abkühlen und filtriert vom ausgeschiedenen Kaliumchlorid ab. Das nach dem Einengen des Filtrats verbleibende Oel wird in 1 000 ml Essigester aufgenommen. Man wäscht zweimal mit je 150 ml 10 %-iger Natriumcarbonat- Lösung und Wasser, trocknet über wasserfreiem Natriumsulfat und destilliert das Lösungsmittel ab. Restmengen an Lösungsmittel werden bei 0,13 mbar (Badtemperatur: 180 °C) abgezogen. Man erhält so 357,2 g (95 % der Theorie) 5-(4-Biphenylyloxy)-3,3-dimethyl-1-penten-4-on vom Schmelzpunkt 42-44 °C.

Beispiel 4

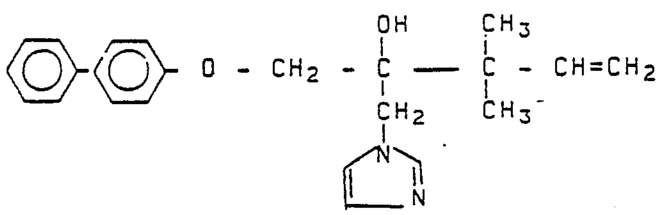

(Verfahren a)

Zu einer siedenden Lösung von 1 g (0,012 Mol) Natriumpropylat und 9 g (0,132 Mol) Imidazol in 50 ml n-Propanol werden innerhalb von 5 Minuten 35,3 g (0,120 Mol) 2-(4-Biphenylyloxymethyl)-2-(3,3-dimethyl-1-propen-3-yl)-oxiran, gelöst in 80 ml n-Propanol, zugegeben. Nach zweitägigem Sieden unter Rückfluß der Lösung wird das Lösungsmittel unter vermindertem Druck abdestilliert, der Rückstand in einem Gemisch aus 150 ml Essigester und 100 ml Dichlormethan aufgenommen und dreimal mit je 50 ml Wasser gewaschen. Nach dem Trocknen der organischen Phase über wasserfreiem Natriumsulfat wird die Lösung unter vermindertem Druck bis zur Trockne eingeengt. Der verbleibende feste Rückstand wird mit 10 ml Essigester versetzt und abfiltriert. Man erhält so 27,2 g (62,5 % der Theorie) 4-(4-Biphenylyloxymethyl)-3,3-dimethyl-4-hydroxy-5-(imidazol-1-yl)-1-penten in Form farbloser Kristalle vom Schmelzpunkt 151-153 °C.

In analoger Weise und entsprechend den erfindungsgemäßen Verfahren (a) und (b) werden die nachfolgenden Verbindungen der allgemeinen Formel

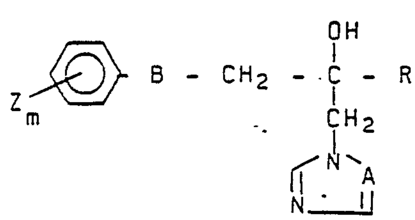

(I)

erhalten :

| Bsp. Nr. | $Z_m$ | B | A | R | Schmelzpunkt (°C) bzw. Brechungsindex $n_D^{20}$ |
|---|---|---|---|---|---|
| 5 | $4-CH_3$ | O | N | $-C(CH_3)_2CH_2F$ | 91-92 |
| 6 | 4-Cl | O | N | $-C(CH_3)_2CH_2F$ | 111-13 |
| 7 | $4-Cl,2-CH_3$ | O | N | $-C(CH_3)_2CH_2F$ | 119-20 |
| 8 | $2,4-Cl_2$ | O | N | $-C(CH_3)_2CH_2F$ | 107-09 |
| 9 | 4-Cl | S | N | $-C(CH_3)_2CH_2F$ | 87 |
| 10 | 2-Cl | S | N | $-C(CH_3)_2CH_2F$ | 1,552 |
| 11 | $3,4-Cl_2$ | S | N | $-C(CH_3)_2CH_2F$ | 1,5710 |
| 12 | 4-Cl | O | N | $-C(CH_2F)_2CH_3$ | 111-12 |
| 13 | $2,4-Cl_2$ | O | N | $-C(CH_2F)_2CH_3$ | 109-10 |
| 14 | $4-Cl,2-CH_3$ | O | N | $-C(CH_2F)_2CH_3$ | 138-39 |
| 15 | $2,4-Cl_2$ | O | N | $-C(CH_3)_2CH_2OCH_3$ | 49-55 |
| 16 | $4-Cl,2-CH_3$ | O | N | $-C(CH_3)_2CH_2OCH_3$ | 1,537 |
| 17 | 4-Cl | O | N | $-C(CH_3)_2CH_2OC_2H_5$ | 69-73 |
| 18 | 4-Cl | O | N | $-C(CH_3)_2CH_2O$ 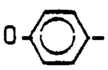 -Cl | 125-26 |
| 19 | 4-F | O | N | $-C(CH_3)_2CH_2OCH_3$ | 1,5152 |
| 20 | $2,4-Cl_2$ | O | N | $-C(CH_3)_2$ 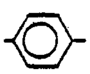 -Cl | 1,5805 |
| 21 | 4-Cl | O | N | $-C(CH_3)_2-CH=CH_2$ | 58-59 |
| 22 | $2,4-Cl_2$ | O | N | $-C(CH_3)_2-CH=CH_2$ | 86-87 |
| 23 | 4-F | O | N | $-C(CH_3)_2-CH=CH_2$ | 66-68 |
| 24 | 4-Cl | $CH_2$ | N | $-C(CH_3)_2CH_2F$ | 110-12 |
| 25 | $2,4-Cl_2$ | $CH_2$ | N | $-C(CH_3)_2CH_2F$ | 88-90 |
| 26 | $2,4-Cl_2$ | $CH_2$ | N | $-C(CH_2F)_2CH_3$ | 97-99 |
| 27 | 4-Cl | $CH_2$ | N | $-C(CH_2F)_2CH_3$ | 122-24 |
| 28 | 4-Cl | $CH_2$ | N | $-C(CH_3)_2CH_2O$ 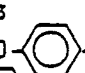 -Cl | 120-21 |
| 29 | 4-Cl | $CH_2$ | N | $-C(CH_3)_2O$ 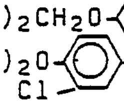 -Cl | 116-18 |
| 30 | 4-Cl | O | CH | $-C(CH_2F)_2CH_3$ | 165-67 |
| 31 | $2,4-Cl_2$ | O | CH | $-C(CH_2F)_2CH_3$ | 148-50 |
| 32 | $4-Cl,2-CH_3$ | O | CH | $-C(CH_2F)_2CH_3$ | 133-35 |
| 33 | 4-Cl | $CH_2$ | CH | $-C(CH_3)_2O$ 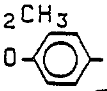 -Cl | 118-20 |
| 34 | 4-Cl | $CH_2$ | CH | $-C(CH_3)_2CH_2O$ 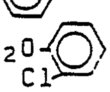 -Cl | 121-23 |
| 35 | $2,4-Cl_2$ | $CH_2$ | CH | $-C(CH_2F)_2CH_3$ | 96-99 |
| 36 | 4-Cl | $CH_2$ | CH | $-C(CH_2F)_2CH_3$ | 128-37 |
| 37 | $2,4-Cl_2$ | $CH_2$ | CH | $-C(CH_3)_2CH_2F$ | 103-06 |
| 38 | $2,4-Cl_2$ | O | CH | $-C(CH_3)_2-CH=CH_2$ | 136-37 |
| 39 | 4-F | O | CH | $-C(CH_3)_2-CH=CH_2$ | 135-36 |

(Fortsetzung)

| Beisp.-Nr. | $Z_m$ | B | A | R | Fp.(°C) bzw. $n_D^{20}$ |
|---|---|---|---|---|---|
| 40 | $4-OCF_3$ | O | N | $-C(CH_3)_2CH_2OCH_3$ | 1,4882 |
| 41 | $4-OCF_3$ | O | N | $-C(CH_3)_2CH_2OC_2H_5$ | 1,4836 |
| 42 | 4-Cl | O | N | $-C(CH_3)_2-O-$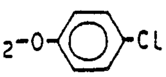 | Harz |
| 43 | 4-F | O | N | $-C(CH_3)_2-O-$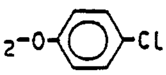 | Harz |
| 44 | $2,4-Cl_2$ | O | N | $-C(CH_3)_2-O-$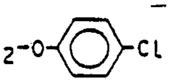 | Harz |
| 45 | 4-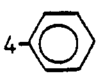 | O | N | $-C(CH_3)_2-O-$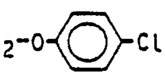 | Harz |
| 46 | 4-Cl | O | N | $-C(CH_3)_2-O-$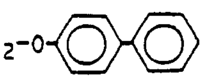 | Harz |
| 47 | $2,4-Cl_2$ | O | N | $-C(CH_3)_2-O-$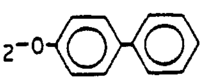 | Harz |
| 48 | $2,4-Cl_2$ | O | N | $-C(CH_3)_2-S-$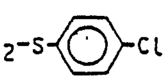 | 115 |
| 49 | 4-Cl | O | N | $-C(CH_3)_2-O-CH_2-$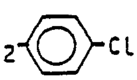 | 133 |
| 50 | $2,4-Cl_2$ | O | N | $-C(CH_3)_2-O-CH_2-$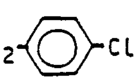 | 102 |

## Verwendungsbeispiele

In den nachfolgenden Verwendungsbeispielen werden die nachstehend angegebenen Substanzen als Vergleichsverbindungen eingesetzt :

(A)

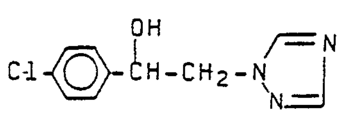

(B)

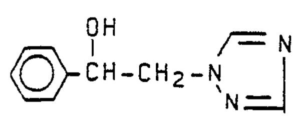

(C) 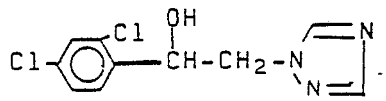

(D) 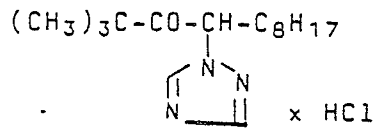

(E) 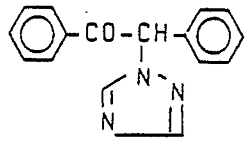

(F) 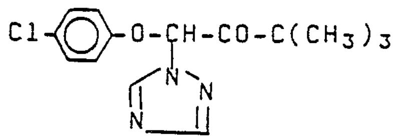

(G) 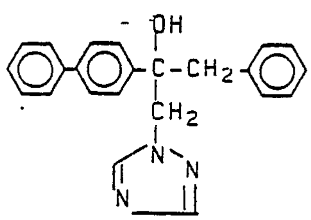

(H) 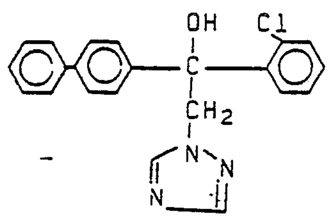

(I) 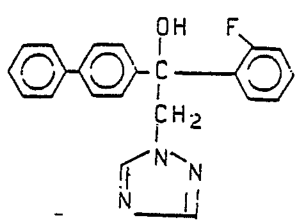

Beispiel A

Erysiphe-Test (Gerste)/protektiv

Lösungsmittel : 100 Gewichtsteile Dimethylformamid

Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung

taufeucht. Nach Antrocknen des Spritzbelages werden die Pflanzen mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 14, 13, 12, 18, 17, 16, 15, 2, 8, 7, 6, 5, 1, 31, 32, 20 und 10.

## Beispiel B

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 14, 13, 12, 17, 16, 15, 6, 5 und 1.

## Beispiel C

Venturia-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 17, 16, 15, 8, 7 und 6.

## Beispiel D

Sphaerotheca-Test (Gurke)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24 °C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 16, 15, 2, 8, 7, 6, 5, 1 und 20.

Beispiel E

Pellicularia-Test (Reis)

Lösungsmittel : 12,5 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25 °C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z. B. die Verbindungen gemäß folgender Herstellungsbeispiele : 5, 6, 7 und 8.

Beispiel F

Wuchshemmung bei Baumwolle

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrolle berechnet. Es bedeuten 100 % Wuchshemmung den stillstand des Wachstums und 0 % ein Wachstum entsprechend dem der Kontrollpflanzen.

Die erfindungsgemäßen Wirkstoffe 1, 5, 6, 7, 12, 16 und 15 zeigen in diesem Test eine bessere Wuchshemmung als die aus dem Stand der Technik bekannten Verbindungen (B), (C), (H) und (I).

Beispiel G

Wuchshemmung bei Sojabohnen

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die erfindungsgemäßen Wirkstoffe 1, 5, 6, 7, 8, 2, 13 und 12 zeigen in diesem Test eine bessere Wuchshemmung als die aus dem Stand der Technik bekannten Verbindungen (B), (C) und (H).

Beispiel H

Wuchsbeeinflussung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen

wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive eine Wuchsförderung gegenüber den Kontrollpflanzen.

Die erfindungsgemäßen Wirkstoffe 20, 1, 5, 6, 7, 2, 16 und 15 zeigen in diesem Test eine stärkere Wuchsbeeinflussung als die aus dem Stand der Technik bekannten Verbindungen (A), (H) und (I).

**Patentansprüche**

1. Substituierte 1-Hydroxyalkyl-azolyl-Derivate der allgemeinen Formel

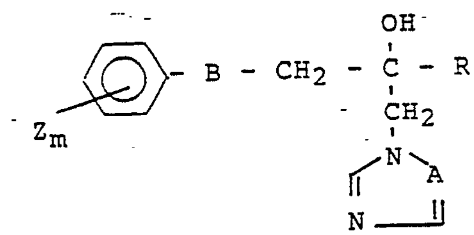

(I)

in welcher

A für ein Stickstoffatom oder die CH-Gruppe steht,

B für Sauerstoff, Schwefel oder die $CH_2$-Gruppe steht,

R für die Gruppierungen

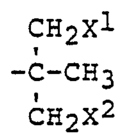

und

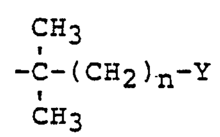

steht, wobei

$X^1$ für Wasserstoff, Fluor, Chlor oder Brom steht,

$X^2$ für Fluor, Chlor oder Brom steht,

Y für Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für gegebenenfalls substituiertes Phenyl, Phenoxy, Phenylthio, Phenylmethoxy und Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl,

n für die Zahlen 0, 1 oder 2 steht,

Z für Fluor, Chlor, Brom, Methyl, tert.-Butyl, Cyclohexyl, Methoxy, Methylthio, Trifluormethyl, Trifluormethoxy, Trifluormethylthio sowie für gegebenenfalls durch Fluor, Chlor und Methyl substituiertes Phenyl, Phenoxy, Benzyl oder Benzyloxy steht, und

m für die Zahlen 0, 1, 2 oder 3 steht, sowie deren Säureadditions-Salze und Metallsalz-Komplexe.

2. Verfahren zur Herstellung von substituierten 1-Hydroxyalkyl-azolyl-Derivaten der allgemeinen Formel

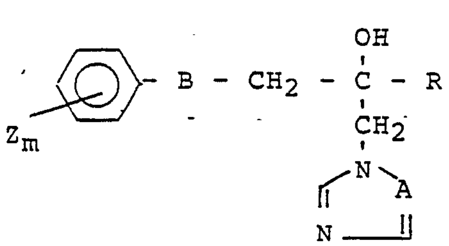

(I)

in welcher

A für ein Stickstoffatom oder die CH-Gruppe steht,

B für Sauerstoff, Schwefel oder die $CH_2$-Gruppe steht,

R für die Gruppierungen

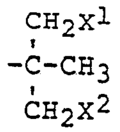 und 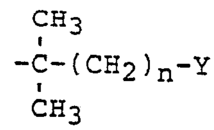

steht, wobei

$X^1$ für Wasserstoff, Fluor, Chlor oder Brom steht,

$X^2$ für Fluor, Chlor oder Brom steht,

Y für Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano, sowie für gegebenenfalls substituiertes Phenyl, Phenoxy, Phenylthio, Phenylmethoxy und Phenylmethylthio steht, wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl,

n für die Zahlen 0, 1, 2 oder 3 steht,

Z für Fluor, Chlor, Brom, Methyl, tert.-Butyl, Cyclohexyl, Methoxy, Methylthio, Trifluormethyl, Trifluormethoxy, Trifluormethylthio, sowie für gegebenenfalls durch Fluor, Chlor und Methyl substituiertes Phenyl, Phenoxy, Benzyl oder Benzyloxy steht, und

m für die Zahlen 0, 1, 2 oder 3 steht,

sowie von deren Säureadditions-Salzen und Metallsalz-Komplexen,
dadurch gekennzeichnet, daß man

a) Oxirane der Formel

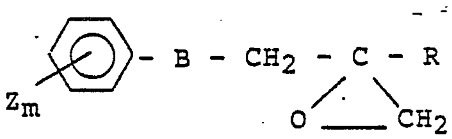

(II)

in welcher B, R, Z und m die oben angegebene Bedeutung haben, mit Azolen der Formel

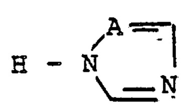

(III)

in welcher A die oben angegebene Bedeutung hat, in Gegenwart eines Verdünnungsmittels und gegebenenfalls in Gegenwart einer Base umsetzt, oder

b) Azolylmethyl-oxirane der Formel

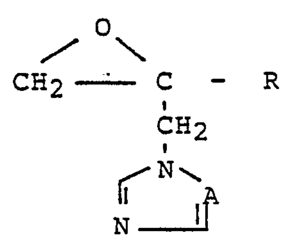

(IV)

in welcher A und R die oben angegebene Bedeutung haben, mit (Thio)Phenolen der Formel

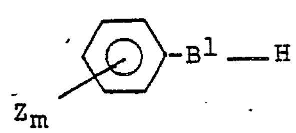

(V)

in welcher

Z und m die oben angegebene Bedeutung haben und

$B^1$ für Sauerstoff oder Schwefel steht, in Gegenwart eines Verdünnungsmittels und gegebenenfalls in Gegenwart einer Base umsetzt, und gegebenenfalls anschließend an die so erhaltenen Verbindungen der Formel (I) eine Säure oder ein Metallsalz addiert.

3. Pflanzenschutzmittel gekennzeichnet durch einen Gehalt an mindestens einem substituierten 1-Hydroxyalkyl-azolyl-Derivat der Formel (I) in Anspruch 1 bzw. einem Säureadditions-Salz oder Metallsalz-Komplex einer Verbindung der Formel (I).

4. Fungizide und das Wachstum von Pflanzen regulierende Mittel, gekennzeichnet durch einen Gehalt an mindestens einem substituierten 1-Hydroxyalkyl-azolyl-Derivat der Formel (I) in Anspruch 1 bzw. einem Säureadditions-Salz oder Metallsalz-Komplex einer Verbindung der Formel (I).

5. Verfahren zur Bekämpfung von Pilzen, dadurch gekennzeichnet, daß man substituierte 1-Hydroxyalkyl-azolyl-Derivate der Formel (I) in Anspruch 1 bzw. deren Säureadditions-Salze oder Metallsalz-Komplexe auf Pilze oder Ihren Lebensraum einwirken läßt.

6. Verfahren zur Regulierung des Pflanzenwachstums, dadurch gekennzeichnet, daß man substituierte 1-Hydroxyalkyl-azolyl-Derivate der Formel (I) in Anspruch 1 bzw. deren Säureadditions-Salze oder Metallsalz-Komplexe auf Pflanzen oder ihren Lebensraum einwirken läßt.

7. Verwendung von substituierten 1-Hydroxyalkyl-azolyl-Derivaten der Formel (I) in Anspruch 1 bzw. von deren Säureadditions-Salzen oder Metallsalz-Komplexen zur Bekämpfung von Pilzen und zur Regulierung des Pflanzenwachstums.

8. Oxirane der Formel

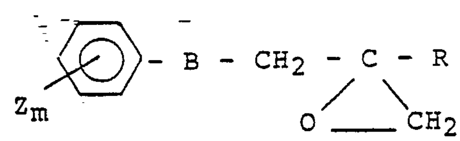

(II)

in welcher Z, R, B und m für die in Anspruch 1 angegebenen Bedeutungen stehen.

## Claims

1. Substituted 1-hydroxyalkyl-azolyl derivatives of the general formula

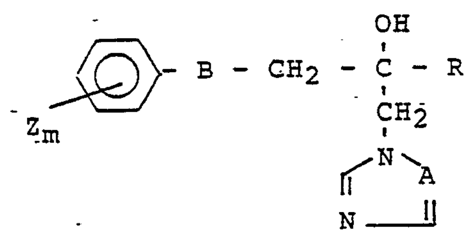

(I)

in which

A represents a nitrogen atom or the CH group,

B represents oxygen, sulphur or the $CH_2$ group,

R represents the groupings

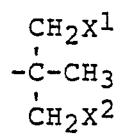

and

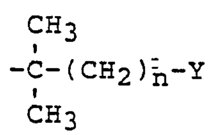

wherein

$X^1$ represents hydrogen, fluorine, chlorine or bromine,

$X^2$ represents fluorine, chlorine or bromine,

Y represents ethyl, propyl, methoxy, ethoxy, methylthio, ethylthio, trifluoromethoxy, trifluoromethylthio, vinyl, methoxycarbonyl, ethoxycarbonyl, cyano and optionally substituted phenyl, phenoxy, phenylthio, phenylmethoxy and phenylmethylthio, the following being mentioned as the phenyl substituents in each case : fluorine, chlorine, methyl, ethyl, methoxy, methylthio, trifluoromethyl, trifluoromethoxy, trifluoromethylthio, dimethylamino, methoxycarbonyl and ethoxycarbonyl,

n represents the numbers 0, 1 or 2,

Z represents fluorine, chlorine, bromine, methyl, tert.-butyl, cyclohexyl, methoxy, methylthio, trifluoromethyl, trifluoromethoxy, trifluoromethylthio and optionally fluorine-, chlorine- and methyl-substituted phenyl, phenoxy, benzyl or benzyloxy, and

m represents the numbers 0, 1, 2 or 3, and their acid addition salts and metal salt complexes.

2. Process for the preparation of substituted 1-hydroxyalkyl-azolyl derivatives of the general formula

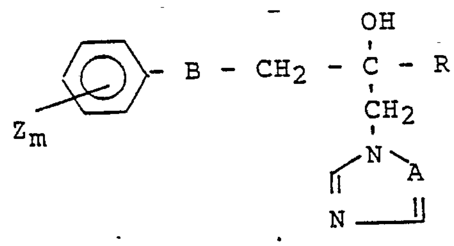

(I)

in which

A represents a nitrogen atom or the CH group,

B represents oxygen, sulphur or the $CH_2$ group,

R represents the groupings

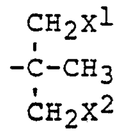

and

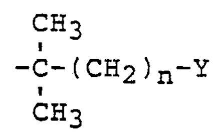

wherein

$X^1$ represents hydrogen, fluorine, chlorine or bromine,

$X^2$ represents fluorine, chlorine or bromine,

Y represents ethyl, propyl, methoxy, ethoxy, methylthio, ethylthio, trifluoromethoxy, trifluoromethylthio, vinyl, methoxycarbonyl, ethoxycarbonyl, cyano, and optionally substituted phenyl, phenoxy, phenylthio, phenylmethoxy and phenylmethylthio, the following being mentioned as the phenyl substituents in each case: fluorine, chlorine, methyl, ethyl, methoxy, methylthio, trifluoromethyl, trifluoromethoxy, trifluoromethylthio, dimethylamino, methoxycarbonyl and ethoxycarbonyl,

n represents the numbers 0, 1, 2 or 3,

Z represents fluorine, chlorine, bromine, methyl, tert.-butyl, cyclohexyl, methoxy, methylthio, trifluoromethyl, trifluoromethoxy, trifluoromethylthio, and optionally fluorine-, chlorine- and methyl-substituted phenyl, phenoxy, benzyl or benzyloxy, and

m represents the numbers 0, 1, 2 or 3, and their acid addition salts and metal salt complexes, characterised in that

a) oxiranes of the formula

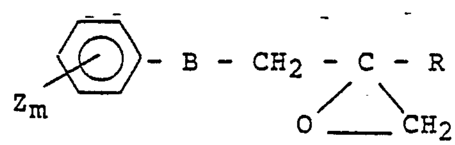

(II)

in which B, R, Z and m have the abovementioned meaning, are reacted with azoles of the formula

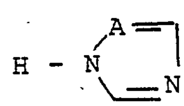

(III)

in which A has the abovementioned meaning, in the presence of a diluent and if appropriate in the presence of a base, or

b) azolylmethyl-oxiranes of the formula

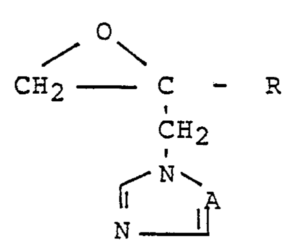

(IV)

in which A and R have the abovementioned meaning, are reacted with (thio)phenols of the formula

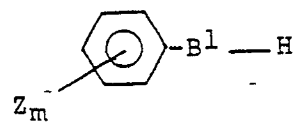 (V)

in which

Z and m have the abovementioned meaning and

$B^1$ represents oxygen or sulphur,

in the presence of a diluent and if appropriate in the presence of a base, and, if desired, an acid or a metal salt is then added on to the compounds of the formula (I) thus obtained.

3. Plant protection agents, characterised in that they contain at least one substituted 1-hydroxyalkyl-azolyl derivative of the formula (I) in Claim 1 or an acid addition salt or metal salt complex of a compound of the formula (I).

4. Fungicidal and plant-growth-regulating agents, characterised in that they contain at least one substituted 1-hydroxyalkyl-azolyl derivative of the formula (I) in Claim 1 or an acid addition salt or metal salt complex of a compound of the formula (I).

5. Method of combating fungi, characterised in that substituted 1-hydroxyalkyl-azolyl derivatives of the formula (I) in Claim 1 or their acid addition salts or metal salt complexes are allowed to act on fungi or their habitat.

6. Method of regulating plant growth, characterised in that substituted 1-hydroxyalkyl-azolyl derivatives of the formula (I) in Claim 1 or their acid addition salts or metal salt complexes are allowed to act on plants or their habitat.

7. Use of substituted 1-hydroxyalkyl-azolyl derivatives of the formula (I) in Claim 1 or their acid addition salts or metal salt complexes for combating fungi and for regulating plant growth.

8. Oxiranes of the formula

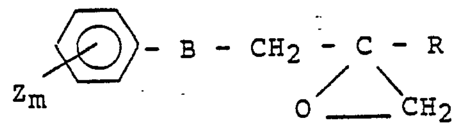 (II)

in which Z, R, B and m represent the meanings given in Claim 1.

## Revendications

1. Dérivés 1-hydroxyalcoyl-azolyliques substitués de formule générale :

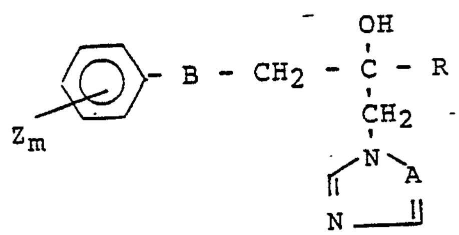 (I)

dans laquelle

A représente un atome d'azote ou le groupe CH,

B de l'oxygène, du soufre ou le groupe $CH_2$,

R les groupements

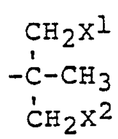 et 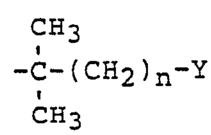

$X^1$ représente de l'hydrogène, du fluor, du chore ou du brome,

$X^2$ du fluor, du chlore ou du brome

Y éthyle, propyle, méthoxy, éthoxy, méthylthio, éthylthio, trifluorométhoxy, trifluorométhylthio, vinyle, méthoxycarbonyle, éthoxycarbonyle, cyano de même que phényle, phénoxy, phénylthio, phénylméthoxy et phénylméthylthio éventuellement substitués, en citant chaque fois comme substituants du phényle : fluor, chlore, méthyle, éthyle, méthoxy, méthylthio, trifluorométhyle, trifluorométhoxy, trifluorométhylthio, diméthylamino, méthoxycarbonyle et éthoxycarbonyle,

n représente les nombres 0, 1 et 2,

Z du fluor, chlore, brome, méthyle, t-butyle, cyclohexyle, méthoxy, méthylthio, trifluorométhyle,

trifluorométhoxy, trifluorométhylthio, de même que phényle, phénoxy, benzyle ou benzyloxy éventuellement substitués par du fluor, du chlore et méthyle,

m les nombres 0, 1, 2 ou 3, de même que leurs sels d'addition d'acides et complexes avec des sels métalliques.

2. Procédé de fabrication de dérivés 1-hydroxyalcoyl-azolyliques substitués de formule générale :

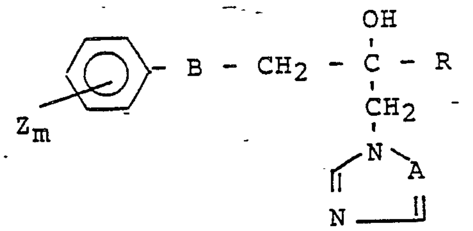

(I)

dans laquelle

A représente un atome d'azote ou le groupe CH,

B de l'oxygène, du soufre ou le groupe $CH_2$,

R les groupements

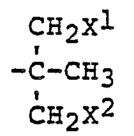

et

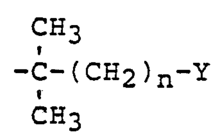

$X^1$ étant de l'hydrogène, du fluor, du chlore ou du brome,

$X^2$ du fluor, du chlore ou du brome,

Y un éthyle, propyle, méthoxy, éthoxy, méthylthio, éthylthio, trifluorométhoxy, trifluorométhylthio, vinyle, méthoxycarbonyle, éthoxycarbonyle, cyano, de même que phényle, phénoxy, phénylthio, phénylméthoxy et phénylméthylthio éventuellement substitués, en citant chaque fois comme substituants du phényle : fluor, chlore, méthyle, éthyle, méthoxy, méthylthio, trifluorométhyle, trifluorométhoxy, trifluorométhylthio, diméthylamino, méthoxycarbonyle et éthoxycarbonyle,

n les nombres 0, 1, 2 ou 3,

Z du fluor, chlore, brome, méthyle, t-butyle, cyclohexyle, méthoxy, méthylthio, trifluorométhyle, trifluorométhoxy, trifluorométhylthio, de même qu'un phényle, phénoxy, benzyle ou benzyloxy éventuellement substitués par du fluor, du chlore et méthyle,

m les nombres 0, 1, 2 ou 3, de même que de leurs sels d'addition d'acides et complexes avec des sels métalliques, caractérisé en ce que

a) on fait réagir des oxiranes de formule :

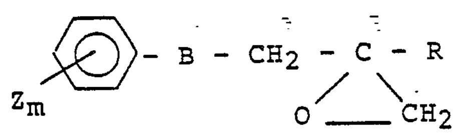

(II)

dans laquelle B, R, Z et m ont les significations indiquées plus haut, avec des azols de formule :

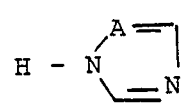

(III)

dans laquelle

A a la signification indiquée plus haut, en présence d'un diluant et éventuellement en présence d'une base, ou bien

b) on fait réagir des azolylméthyl-oxiranes de formule :

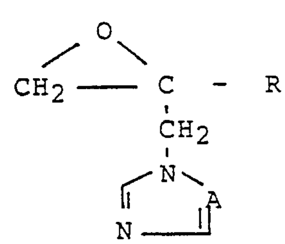

(IV)

dans laquelle A et R ont les significations indiquées plus haut, avec des (thio)phénols de formule :

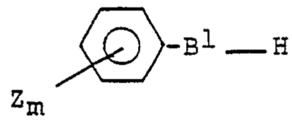

(V)

dans laquelle

Z et m ont les significations indiquées plus haut et

$B^1$ représente de l'oxygène ou du soufre, en présence d'un diluant et éventuellement en présence d'une base, et en ce que par la suite on fixe éventuellement sur les composés ainsi obtenus de formule (I) un acide ou un sel métallique.

3. Agents de protection des plantes caractérisés par une teneur en au moins un dérivé 1-hydroxyalcoyl-azolylique substitué de formule (I) selon la revendication 1 ou en un sel d'addition d'acide ou complexe avec un sel métallique d'un composé de formule (I).

4. Agents fongicides et régulateurs de la croissance des plantes, caractérisés par une teneur en au moins un dérivé 1-hydroxyalcoyl-azolylique substitué de formule (I) selon la revendication 1 ou en un sel d'addition d'acide ou complexe avec un sel métallique d'un composé de formule (I).

5. Procédé pour combattre les champignons, caractérisé en ce qu'on fait agir des dérivés 1-hydroxyalcoyl-azolyliques substitués de formule (I) selon la revendication 1 ou leurs sels d'addition d'acides ou complexes avec des sels métalliques sur les champignons ou sur leur espace vital.

6. Procédé de régulation de la croissance des plantes, caractérisé en ce qu'on fait agir des dérivés 1-hydroxyalcoyl-azolyliques substitués de formule (I) selon la revendication 1 ou leurs sels d'addition d'acides ou complexes avec des sels métalliques sur des plantes ou sur leur espace vital.

7. Utilisation de dérivés 1-hydroxyalcoyl-azolyliques substitués de formule (I) selon la revendication 1 ou de leurs sels d'addition d'acides ou complexes avec des sels métalliques pour combattre les champignons et pour régler la croissance des plantes.

8. Oxiranes de formule :

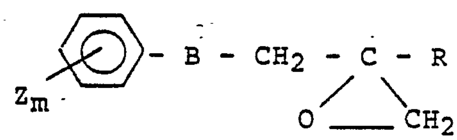

(II)

dans laquelle Z, R, B et m ont les significations indiquées à la revendication 1.